# NEW EUROPEAN PATENT SPECIFICATION

(11) **EP 3 236 954 B2**
(45) Date of publication and mention of the opposition decision: **04.09.2024**
(45) Mention of the grant of the patent: 23.03.2022
(21) Application number: 15874302.1
(22) Date of filing: 22.12.2015
(51) Int. Cl.: A61K 9/28, A61K 9/30, A61K 9/34

(54) **ENTERIC FILM COATING COMPOSITIONS, METHOD OF COATING, AND COATED FORMS**
ENTERALE FILMBESCHICHTUNGSZUSAMMENSETZUNGEN, VERFAHREN ZUR BESCHICHTUNG UND ÜBERZOGENE ARZNEIFORMEN
COMPOSITION DE PELLICULAGE ENTÉRIQUE, PROCÉDÉ D'ENROBAGE, ET FORMES ENROBÉES

(30) Priority: 23.12.2014 US 201462124590 P
(43) Date of publication of application: 01.11.2017
(73) Proprietor: DuPont Nutrition USA, Inc., Wilmington DE 19805 (US)
(72) Inventor: YANG, Hui, S., Plainsboro, NJ 08536 (US); MA, Hua, Skillman, NJ 08558 (US); SESTRICK, Michael, Yardley, PA 19067 (US)
(74) Representative: f & e patent
(86) International application number: PCT/US2015/067370
(87) International publication number: WO 2016/106315

(56) References cited:
- WO-A1-2012/022498
- WO-A1-2012/131476
- WO-A1-2012/131476
- WO-A1-2014/136857
- WO-A1-2014/136857
- FR-A1- 2 934 467
- US-A- 5 733 575
- US-A1- 2004 062 855
- US-A1- 2005 106 233
- US-A1- 2011 059 165
- VESKI P ET AL: "SODIUM ALGINATES AS DILUENTS IN HARD GELATIN CAPSULES CONTAINING IBUPROFEN AS A MODEL DRUG", PHARMAZIE, GOVI VERLAG PHARMAZEUTISCHER VERLAG GMBH, DE, vol. 48, no. 10, 1 October 1993 (1993-10-01), pages 757 - 760, XP000398955, ISSN: 0031-7144
- VELINE VALERIA LIEW ET AL.: "Evaluation of sodium alginate as drug release modifier in matrix tablets", INTERNATIONAL JOURNAL OF PHARMACEUTICS, vol. 309, 2006, pages 25 - 37, DOI: 10.1016/j.ijpharm.2005.10.040
- VESKI P. ET AL.: "Sodium Alginates as diluents in hard gelatin capsules containing ibuprofen as a model drug", PHARMAZIE, vol. 48, no. 10, 1993, pages 757 - 760
- MIKINORI U. ET AL.: "Biological Activities of Alginate", ADVANCES IN FOOD NUTRITION RESEARCH, vol. 72, 2014, pages 95 - 112
- EFA, April 29, 5, Rina Chokshi, FMC.pdf

## Description

### CROSS REFERNCE TO RELATED APPLICATIONS

The present application claims the benefit of U.S. Provisional Application No. 62/124,590, filed December 23, 2014.

### FIELD OF THE INVENTION

This invention relates to the field of enteric film coatings including dry powder formulations and aqueous solutions used in making enteric film coatings; enteric coated oral dosage forms of orally ingestible active ingredients including capsules, tablets, and the like, for preventing release of the ingredients of the active ingredient in the gastric juices of the stomach, and for releasing the ingredients after passing the stomach, for example in the intestines; and methods of making the dry powder formulations, aqueous solutions, and enteric coated oral dosage forms. The invention provides a food safe, plant based, water soluble, dry powder formulation and aqueous enteric coating solutions made therefrom that may be used in coating oral dosage forms with an intestinally soluble coating that is insoluble in the gastric juices of the stomach and methods of making the dry powder formulations and aqueous enteric coating solutions. The invention also provides various oral dosage forms that are coated with an enteric film coating using the aqueous enteric coating solutions and methods of coating oral dosage forms.

### BACKGROUND OF THE INVENTION

Enteric coating materials are material types that are acid resistant, protecting and preventing the coated oral dosage form from a releasing of the contents into the stomach. However, these coatings dissolve or disintegrate in the neutral or mildly alkaline conditions that are encountered beyond the stomach. Enteric coatings can also be used for avoiding irritation of or damage to the mucous membrane of the stomach caused by substances contained in the oral dosage form, and for counteracting or preventing formation or release of substances having an unpleasant odor or taste in the stomach. Finally, such coatings can be used for preventing nausea or vomiting on intake of the coated oral dosage form. Many enteric coating have been approved for pharmaceutical use but few are approved for food use. Many dietary supplements and health foods are available in oral dosage forms which may benefit from enteric coatings. However without approval for food use, enteric coating compositions may not be used on oral dosage forms in nutraceutical applications. It would be useful to provide an enteric composition made from materials that are approved for pharmaceutical, nutriceutical, veterinary, and food use so that such coatings may also be used on non-pharmaceutical dosage forms.

Many of the previously available enteric materials required the use of organic solvents. The use of organic solvents presents numerous problems including increased pollution greater danger of fire and explosion. Expensive equipment and training of workers handling the organic solvents is required to mitigate these risks. Aqueous enteric coating materials have also been proposed but many lack adequate enteric performance. Therefore, it would be beneficial to provide an aqueous enteric composition that is able to provide adequate enteric coating performance.

Many of the previously available enteric materials included phthalates. Enteric coating utilizing phthalates include hydroxypropyl methylcellulose phthalate (HPMCP), cellulose acetate pthalate (CAP), and polyvinyl acetate phthalate (PVAP). However, phthalates have disadvantages in that they require additional undesired processing steps. HPMCP for example, generally requires neutralization with a base, such as ammonia, to increase solubility and must also undergo an acid treatment after spraying in order to provide enteric functionality. The addition of ammonia results in an unpleasant smell. Furthermore, the acid treatment step requires the use of a strong acid with a pH in the range of 1 - 3. Therefore, it is desirable and there is a need for enteric film coating compositions having enteric polymers other than phthalates.

Alginates, derived from, inter alia, brown seaweeds (*Phaeoyhyceae sp.*) are linear unbranched chemical polymers containing (1-4)-linked β-D-mannuronic acid (M) and C-5 epimer α-L-guluronic acid (G) residues. Alginates consist of homopolymeric blocks of consecutive G residues, consecutive M residues, or alternating M and G residues, for example, MMMM, GGGG, and GMGM. Alginates are widely used in the food industry to increase viscosity, form hydrogels, and also used as emulsifiers. Alginates have also been used in capsules. Alginates are generally useful in the form of alginic acid or salts thereof. The number of G residues with respect to the sum of G and M residues is known as G content. Similarly the percent content of M residues is known as M content, such that the G content and M content account for 100%. The content of the G and M residues may also be expressed as a M/G ratio. M/G ratios may be determined by NMR or IR spectroscopy using methods known in the art. The G content in major algal alginates generally is between roughly 30 % and 70%. However, different seaweeds yield alginates that differ in monomeric composition. A wide range of alginates are commercially available including for example the PROTANAL and MANUCOL lines of alginates (FMC BioPolymer, Philadelphia, PA). Gels and films made from alginate possessing a relatively high amount of G blocks are mechanically stronger than those made from alginate possessing a relatively high amount of M blocks. Accordingly, gels and films made of high-M alginates generally have a poor mechanical strength compared to gels and films made of high-G alginates. Furthermore, increasing the concentration of an alginate having a high M content in the film coating would be expected to unacceptably increase process viscosity and create very difficult processing conditions, while decreasing the molecular weight of such an alginate would be expected to undesirably result in further weakening the (already very weak) film even at high concentrations. While sodium alginate is water soluble at neutral pH but insoluble at low pH it has not able to prove adequate enteric performance when used as the only enteric polymer.

WO 2012/022498 discloses a gastric resistant coating layer comprising 10 to 100 % by weight of one or more salts of alginic acid with a viscosity of 30 to 720 mPa·s of a 1 % aqueous solution.

Therefore there is a need for a food safe, plant based, water soluble, phthalate-free dry powder formulation and aqueous enteric coating solutions made therefrom that can provide adequate enteric coating performance that is suitable for pharmaceutical, nutraceutical, veterinary, and food use.

### SUMMARY OF THE INVENTION

It is an object of this invention is to provide a food safe, water soluble dry powder formulation that is useful for making an aqueous enteric coating solution and/or enteric film coating comprising an alginate and a water soluble film formation polymer having a 10:1 to 1:3 ratio of alginate to film formation polymer by weight, the alginate is water soluble at 10% solids and has a G content of 20% to 40% and a viscosity of 3-20 mPa·s at 1% solids (measured at 20°C+/- 2°C using a Brookfield viscometer spindle #62), the film formation polymer is water soluble at 10% solids, and whereby when the enteric film coating dry powder formulation is used for an enteric coating of an oral dosage form containing an active ingredient, the active ingredient is not released into a medium of 0.1 N HCl at about 37°C for up to one hour and thereafter about 90% or more of the active ingredient is released within two hours in a phosphate buffer medium having pH 7.2 at about 37°C. Preferably, the active ingredient is not released into a medium of 0.1 N HCl at about 37°C for up to one hour and thereafter about 90% or more of the active ingredient is released in less than one hour or, more preferably, less than 30 minutes in a phosphate buffer medium having pH 7.2 at about 37°C.

Preferably the dry powder formulation is substantially phthalate-free.

The enteric polymer is an alginate. Preferably the enteric polymer is the monovalent salt form of alginate, for example sodium alginate or potassium alginate. Most preferably the enteric polymer is sodium alginate. Preferably the film formation polymer is made from one or more plant based material. More preferably, the film formation polymer is polyvinyl alcohol-polyethylene glycol graft copolymer (commercially available from BASF as KOLLICOAT IR), low viscosity Carrageenan (FMC BioPolymer, Philadelphia, PA), hydroxypropyl methylcellulose (HPMC), acacia gum, or a low viscosity modified starch. Even more preferably, the film formation polymer is HPMC, acacia gum, or a low viscosity modified starch. Low viscosity modified starches may be made from tapioca starch, corn starch, pea starch, or other suitable food starches. Preferably the viscosity of the film formation polymer is 100 to 1000 cps at 10% solids and more preferably 100 to 500 cps at 10% solids. 1 cps = 1 mPa·s.

It is an object of this invention to provide aqueous enteric coating solutions using any of the above mentioned dry powder formulations for use in providing an enteric film coating with adequate enteric performance for pharmaceutical, nutraceutical, veterinary, and food applications. The aqueous enteric coating solution is comprised of any of the above mentioned dry powder formulations dissolved in water and optionally a plasticizer, an antifoaming agent, adhesion enhancing agent, colorant, and anti-tacking agent.

Preferably, the plasticizer is glycerin. Preferably, the antifoaming agent is simethicone. Preferably the aqueous enteric coating solution has a viscosity of 50 to 2000 cps, more preferably 200 to 1000 cps, most preferably 400 to 600 cps. The percent solids of the aqueous enteric coating solution is preferably 2% to 30%, more preferably 3% to 20%, even more preferably 5% to 20%, and most preferably 10% to 20%.

It is also an object of this invention to provide oral dosage forms containing an active ingredient coated using any of the above mentioned aqueous enteric coating solutions to provide an enteric film coating having adequate enteric performance on the oral dosage form.

Preferably after the enteric coated oral dosage form is stored at 20 degrees Celsius for six weeks the active ingredient contained in the enteric film coated oral dosage form is not released when placed into a medium of 0.1 N HCl at about 30 degrees Celsius for up to one hour and thereafter about 90% or more of the active ingredient is released within two hours in a phosphate buffer medium having pH 7.2 at about 37 degrees Celsius (the phosphate buffer can be 0.05M potassium phosphate monobasic adjusted to pH 7.2 by addition of sodium hydroxide). Preferably the oral dosage form is a tablet or capsule, and more preferably a tablet or soft capsule, and most preferably a soft capsule. Preferably the active ingredient is a pharmaceutical, nutraceutical, veterinary product, or food product. More preferably the active ingredient is fish oil, garlic, Probiotic, Lumbrokinase, S-Adenosyl Methionine (SAM-e), Nattokinase, a non-steroidal anti-inflammatory drug, or iron supplement. Most preferably the active ingredient is fish oil.

Additional aspects of the invention include methods of making the dry powder formulation, the aqueous enteric coating solutions, and methods of coating pharmaceutical, nutraceutical, veterinary, and food products, in particular oral dosage forms, with the inventive aqueous enteric coating solutions. Preferred methods of applying the enteric film coating include spraying, dipping, and tumble coating.

### DETAILED DESCRIPTION

The inventors have discovered that the certain combinations of film formation polymers and alginates are able to provide a dry powder formulation which is food safe, plant-based, phthalate-free, and water soluble and when dissolved in water able to be used to coat oral dosage forms to provide an enteric film coating. In particular, the inventors have discovered that combinations of certain film formation polymers and alginates with low to medium G content are able to provide coating with adequate enteric properties while the film formation polymers alone, alginate alone, or the combinations using an alginate with high G content are not able to match the same level of enteric performance or are too viscous to be commercially applicable. Furthermore, the inventors have identified combinations of film formation polymers and alginates which have viscosities that allow for use in spray coating as well as other coating methods. One advantage of this system is that the water solubility of the dry powder formulations makes them easier to process and do not require additional steps or components to enhance solubility. In particular, the inventive products and methods do not require the use of ammonia or strong acids. Another advantage is that these combinations are able to be used with plasticizers without destroying their enteric performance.

According to one embodiment of the present invention, there is provided a dry powder formulation comprising a water soluble film formation polymer and a water soluble enteric polymer as defined in claim 1.

Acceptable film formation polymers able to provide sufficient mechanical strength to allow for film formation. An acceptable viscosity range of the film formation polymer is 100 to 1000 centipoise (cps) at 10% solids. Preferably the viscosity range is 100 to 500 cps at 10% solids. Examples of suitable water soluble film formation polymers include polyvinyl alcohol-polyethylene glycol graft copolymer (commercially available from BASF as KOLLICOAT IR), low viscosity Carrageenan (FMC BioPolymer, Philadelphia, PA), HPMC, acacia gum, and low viscosity modified starch. Low viscosity modified starches may be made from tapioca starch, corn starch, pea starch, or other suitable food starches. Examples of suitable low viscosity modified starch include CRYSTAL TEX 627M (Ingredion Inc., Bridgewater, NJ), Instant PURE-COTE B792 (Grain Process Corp., Muscatine, IA), LYCOAT RS720 and RS780 (Roquette, Lestrem, France). Examples of commercially available acacia gum include ENCAPSIA (Nexira, Somerville, NJ). The water soluble enteric polymers are alginates. Preferably, the alginate salts are monovalent salts and more preferably the alginate is in the form of sodium alginate or potassium alginate. Most preferably is sodium alginate, for example MANUCOL LD, PROTANAL CR 8133, and PROTANAL GP3350 (FMC BioPolymer). The G content is 20% 40%. Accordingly the M content is 60% to 80%. Each of the components of the dry powder formulation is preferably mixed together in a dry powder form.

According to one embodiment of the present invention, the water soluble film formation polymer is a low viscosity modified plant starch. In these embodiments, little to no foaming was observed when the formulations where processed. This provides an advantage over other film formation polymers including HPMC in that less antifoaming agents is required and preferably no antifoaming agent is used. Furthermore, the combination of low viscosity modified plant starch and alginate resulted in a homogeneous transparent film with a broader range of plasticizers as compared to the combination of alginate and HPMC. Finally, the combination of low viscosity modified plant starch and alginate provided improved adhesion properties of the resulting coating as compared to HPMC and alginate.

According to another embodiment of the present invention, there is provided an aqueous enteric coating solutions for use in providing an enteric film coating comprising any one of the above mentioned dry powder formulations, and water, and optionally an antifoaming agent. Suitable antifoaming agents include without limitation silicones, silanes, polydimethylsiloxane (PDMS), hydrophobic silica, dimethicones, and simethicones. A preferred antifoaming agent is 30% Simethicone USP, for example DOW CORNING Q7-2587. Preferably the water is deionized water. The aqueous enteric coating solution has a viscosity of 50 to 2000 cps, preferably the viscosity is 100 to 1000 cps, and most preferably the viscosity is 100 to 800 cps.

According to another embodiment of the present invention, the aqueous enteric coating solution further comprises a plasticizer. Suitable plasticizers include without limitation glycerol, sorbitol, maltitol, triethyl citrate, and polyethylene glycols. A preferred plasticizer is glycerol.

According to another embodiment of the present invention, there is provided an enteric film coated oral dosage form wherein an active ingredient is provided as an oral dosage form and is coated using any one of the above mentioned aqueous enteric coating solutions. Preferred oral dosage forms include an encapsulated powder, tablet, caplet, microcapsule, or capsule. More preferably, the oral dosage form is a softgel capsule or tablet. Most preferably the oral dosage from is a softgel capsule. The oral dosage form may contain any components that are commonly used in such dose forms, such as pharmaceutical active agents, nutraceuticals, veterinary products, food products, and additional excipients. Suitable pharmaceutical active agents include without limitation an oil-soluble, oil-insoluble, watersoluble, and water-insoluble drug. For softgel capsule and tablet oral dosage forms, suitable pharmaceutical active agents include oil-soluble, oil-insoluble, and water insoluble drugs. A preferred pharmaceutical active agent is an omega-3 fatty acid. Suitable nutraceuticals include oils, probiotics, proteins, enzymes, herbs, roots, leafs, fruits, flowers, grasses, barks, fruit peels, minerals or trace minerals in ionic or elemental form, such as calcium, magnesium, zinc, selenium and iron, and combinations thereof. For example, the nutraceutical is Lumbrokinase, S-Adenosyl Methionine (SAM-e), or Nattokinase. Preferred nutraceuticals are oils; more preferably an oil that is high in omega-3 fatty acids; and most preferably fish oil. The enteric film coating provided on the oral dosage form has a thickness of 500 microns or less. Prior to being coated the oral dosage form may have immediate release, controlled, delayed release capabilities or can be released upon activation by a known event, condition, or process. Preferably the oral dosage form has an immediate release profile prior to being coated. Preferably it is beneficial upon ingestion of the oral dosage form to prevent the release of at least one component into the stomach containing the component(s). More preferably at least one component in the enteric coated oral dosage form is susceptible to causing irritation of or damage to the mucous membrane of the stomach or forming or releasing substances having an unpleasant odor or taste in the stomach. Most preferably at least one component tends to form or release substances having an unpleasant odor in the stomach when ingested without an enteric coating, for example, garlic or fish oil. Preferably the thickness of the enteric film coating is 20 to 200 microns. Most preferably the thickness of the enteric film coating is 30 to 90 microns. A suitable amount of weight gain due to coating with the enteric film coating is 2% to 50. Preferably the weight gain is 2 to 15%.

Processes are well known in the industry as methods of making oral dosage forms. The fill materials for the soft capsules can be any fill materials commonly used in such dosage forms. Generally, the fill materials can be liquids (including emulsions) or solids such as powders. The fill materials can be a pharmaceutical ingredient, nutraceutical ingredient, veterinary ingredient, food, etc. For tablet and softgel capsule oral dosage forms, the fill materials are water-insoluble.

Definitions of "disintegration", "disintegrates", "rupture", "dissolution", "immediate release", "delayed release", "enteric", and "enteric coating" can be found in the U.S. Pharmacopeia 32 (USP32). Similarly, the methods for measuring disintegration and dissolution are as described in the examples and in USP 32, chapter <2040>, including the chapter about 'Delayed-release (Enteric coated) tablets. As used herein the term "phthalate-free" means that the composition contains no more than trace amounts of phthalates. As used herein the term "substantially phthalate-free" means that the composition contains less than 10% phthalates by weight. In embodiments of the present invention, the dry powder formulation contains less than 10%, 5%, or 1% phthalates by weight. In embodiments of the present invention, the amount of phthalates contained in the dry powder formulation is 0% by weight. In embodiments of the present invention, the aqueous enteric coating solution contains less than 10%, 5%, or 1% phthalates by weight. In embodiments of the present invention, the amount of phthalates contained in the aqueous enteric coating solution is 0% by weight.

As used herein the term "enteric film coating" means an enteric coating with a thickness of 500 microns or less. In accordance with the definition found in the USP32, the term enteric coating is used herein as meaning an acid resistant layer or covering of an oral dosage form containing an active ingredient, that is able to protect and prevent the coated oral dosage from for a period of time from releasing the contents of the active ingredient into the stomach, and is thereafter able to dissolve or disintegrate in the neutral or mildly alkaline conditions thereby releasing the contents of the active ingredient in a portion of the gastrointestinal tract beyond the stomach. In this manner, an enteric coating permits transit through the stomach to the small intestine before the active ingredient is released.

According to another embodiment of the present invention, there is provided a method of making the above mentioned aqueous enteric coating solutions comprising dissolving a plasticizer and optionally an antifoaming agent in water by mixing under agitation, adding an amount of film formation polymer and an amount of an enteric polymer to the mixture under agitation, and mixing the resulting mixture under high agitation. Optionally, the film formation polymer and enteric polymer are mixed together to make a dry powder formulation prior to being added to the mixture of plasticizer, antifoaming agent, and water.

According to another embodiment of the present invention, there is provided a method of coating an oral dosage form using the inventive aqueous enteric coating solutions to provide an enteric film coating to the oral dosage form comprising spraying, dipping, tumble coating using a coating pan, or coating using a fluid bed coater. These devices and their settings are well known to one of ordinary skill in the art and may be used for coating oral dosage forms using the inventive aqueous enteric coating solutions.

In one embodiment of the present invention, oral dosage forms were spray coated using a Thomas Accela-Cota Compu-Lab spray coater, under the settings listed in Table 1. (25.5 psi = 1.76 bar; 280 CFM = 0.132 m³/s; 3.5 inch = 8.9 cm). A person of ordinary skill in the art will understand how to adjust the spray coating settings in order to adjust to the size of the coating batch or oral dosage form to be coated.

**Table 1. Spray coating settings.**

| **Setting** | **Value** |
|---|---|
| Atom Air | 25.5 psi |
| Pump Rate | 12 ml/minute |
| Inlet Temperature | 45°C |
| Outlet Temperature | 35°C to 38°C |
| Air flow volume | 280 CFM |
| Pan Speed | 9 RPM |
| Spray Distance | 3.5 inch |
| Gun Angle | 45° |
| Soft Gel Size | 7 mm |

All viscosities referred to herein may be measured using a Brookfield Viscometer at appropriate spindle and speeds in water at room temperature such as 18-25°C or at 20°C+/- 2°C. For example, all viscosities herein may be measured using a Brookfield Viscometer, spindle #62, 12 rpm, at 20°C+/- 2°C.

Nonlimiting embodiments of the present invention also include other suitable components in the formulation, solutions, or oral dosage form. Other suitable components include, without limitation, conventional excipients such as binders (such as microcrystalline cellulose), disintegrants, diluents, lubricants, glidants, matrix formers, emulsifying-solubilizing agents, sweetening agents, antimicrobial preservatives, dyes; colorants and pigments such as titanium dioxide and calcium carbonate; stabilizing polymers, such as chitosan; cellulose gums, carrageenan, additional forms of alginates, propylene glycol alginate, gellan, xanthan gum, locust bean gum, guar, pectins, gum arabic, gum tragacanth, sodium-carboxymethylcellulose, alkyl cellulose ethers other than HPMC, hydroxypropylcellulose, hydroxyethylcellulose and methylcellulose and agar-agar; preservatives such as lower alkylparabens, sorbic acid, potassium sorbate, benzoic acid, sodium benzoate, or esters of hyrdoxybenzoic acid, and benzyl alcohol; antioxidants such as ascorbic acid, ascorbyl palmitate, sulfites, L-tocopherol, butylated hydroxyanisole and propyl gallate; disintegrating compounds, and other components. The amount required for these other suitable components will depend on the component, the composition of the product, storage conditions, and are known to those with the necessary skill in the art.

The present invention is now described in more detail by reference to the following examples, but it should be understood that the invention is not construed as being limited thereto. Unless otherwise indicated herein, all parts, percents, ratios and the like are by weight.

### Method for Preparing the Aqueous Enteric Coating Solutions

A plasticizer (glycerol) and optionally an antifoaming agent (30% Simethicone, DOW Q7-2587, Dow) were added to deionized water according to the amounts set forth in table 2 while under agitation at about 300 RPM using a mixer (Heidolph RZR 2021). Once the plasticizer and optional antifoaming agent were fully dissolved or dispersed, the enteric polymer (low viscosity alginate, Manucol LD, FMC BioPolymer) and film formation polymer (low viscosity HPMC, HPMC E3 LV, FMC BioPolymer) were slowly added according to the amounts set forth in table 2 while maintaining agitation at about 300 RPM into the vortex created by the agitated water to avoid lumping. Premixing the enteric polymer and film formation polymer is optional and may help to enhance dissolution. Once all the enteric polymer and film formation polymer were added, the agitation speed was increase to about 450 RPM and mixed for another 2 hours to ensure complete hydration. The solution was then filtered through a 106 micron screen (140 mesh sieve) before coating. After coating, the coatings are not cross-linked. Alginate based coatings are usually cross-linked using a treatment step for example by spraying, dipping, or washing with calcium salt. As described in the examples below, the coatings of the present invention were able to provide adequate enteric coating performance even without crosslinking step(s).

**Table 2. Solution formulation**

| | **Weight (g)** | **% Solids** |
|---|---|---|
| Enteric Polymer (Manucol LD) | 4.20 | 4.20 |
| Film Formation Polymer (HPMC E3 LV) | 4.20 | 4.20 |
| Plasticizer (glycerol) | 1.68 | 1.68 |
| Antifoaming Agent (DOW Q7-2587) (optional) | 0.10 | 0.10 |
| DI Water | 89.82 | |
| Total | 100.00 | 10.18 |

### Method for Testing Enteric Performance

In accordance with the aspects of this invention, the enteric coating has adequate enteric coating performance if the coating remains substantially insoluble in Simulated Gastric Fluid (SGF) at 37±2° C for at least one hour and thereafter disintegrates or ruptures within two hours in Simulated Intestinal Fluid (SIF) at 37±2° C. SGF is a medium of 0.1 N HCl. SIF is a phosphate buffer medium with pH 7.2. Itis made by dissolving 136.0 g KH₂PO₄ (Merck, Lot A585477) in 5 L of deionized water, adding 61.6 ml 5N NaOH in 10 L deionized water, and diluting to a final volume of 20 L. More specifically, enteric coated softgel capsule oral dosage forms were tested for rupture using a Dr. Schleuniger Pharmatron tablet disintegration tester according to standard method USP 32 chapter <2040>. A similar method may be used for disintegration testing of enteric coated tablets. One (1) softgel capsule was placed in each of the six tubes of the basket. The apparatus was then operated using SGF maintained at 37°C as the immersion fluid. After 1 hour of operation in SGF, the basket was lifted from the fluid, and the observe softgel capsules were observed to check for evidence of disintegration, cracking, rupture, leaking, or softening. Next, the apparatus was operated using SIF, maintained at 37°C, as the immersion fluid until all the softgel capsules completely empty and the enteric film coating completely dissolves. The times taken for each capsule to completely empty and to completely dissolve were recorded. If 1 or 2 capsules fail to completely empty within 2 hours, repeat the test on 12 additional capsules. Not fewer than 16 of the total of 18 tablets tested should be completely empty within 2 hours.

### EXAMPLE 1

### Enteric Performance of Enteric Film Coated Softgel Capsules

An aqueous enteric coating solution was prepared according to the formulation in Table 3. The formulation had a viscosity of 795 cps at 9.9% solids. S19 SeaGel^{®} softgels (1.3 cm x 0.95 cm (0.5" x 0.375") diameter, available from FMC Corp., Philadelphia, PA) were filled with oil and spray coated. The resulting coating had a thickness of 69 microns to 74 microns and the coated capsules were 7.4% heavier. After coating, the capsules were tested for enteric performance according to the method described above. After 1 hour in SGF, 6/6 of the coated capsules remained intact. Afterword, the coated soft gels were placed in SIF. After 1 minute all of the coated capsules began leaking oil and all were completely emptied by 5 minutes. All the capsule shells were completely dissolved within 11 minutes. These results suggest that the combination of low viscosity alginate with low G content and a film formation polymer when used with a plasticizer and an antifoaming agent is able to provide an enteric film coating with adequate enteric performance. In contrast, when similar coatings were prepared without film formation polymers the coatings were not able to remain intact in SGF for 1 hour (see, Comparative Examples 8). In other alternative coating formulation, high G content alginate was used with and without a film formation polymer. In both instances, coatings made with high G content alginate failed to remain intact for more than 15 minutes in SGF (see, Comparative Examples 9, 10, and 11).

**Table 3. Softgel Capsule Enteric Film Coating Formulation**

| | **Weight (g)** | **% Solids** |
|---|---|---|
| Enteric Polymer (Manucol LD) | 4.5 | 4.5 |
| Film Formation Polymer (HPMC E3 LV) | 4.5 | 4.5 |
| Plasticizer (glycerol) | .09 | 0.9 |
| Antifoaming Agent (DOW Q7-2587) | 0.10 | 0.10 |
| DI Water | 90.0 | |
| Total | 100.00 | 10.0 |

### EXAMPLE 2

### Enteric Performance of Enteric Film Coating using KOLLICOAT IR

An aqueous enteric coating solution was prepared using a 2:1 ratio of Manucol LD to KOLLICOAT IR according to the formulation in Table 4. The formulation had a viscosity of 1182 cps at 9% solids and coated onto S23 gelatin soft capsules (2.5 cm x 0.83 cm (1.0" x 0.33") diameter). The resulting coated capsules had a weight gain of 10.9%. After coating, the capsules were tested for enteric performance according to the method described above. 5 capsules passed 2 hrs in SGF and 1 ruptured in 60 minutes. The intact coated soft gels were placed in SIF. After 7 minute the 5 remaining coated capsules completely emptied and completely dissolved within 15 minutes. In another experiment a 1:1 ratio of Manucol LD to KOLLICOAT IR was used to coat capsules, 5/6 of these coated capsules remained intact with 1 coated capsule rupturing after 45 minutes. These results show that the combination of low viscosity alginate with low G content and KOLLICOAT IR when used an antifoaming agent is able to provide an enteric film coating with adequate enteric performance and that adjusting the ratio of Manucol LD to KOLLICOAT IR can improve enteric performance.

**Table 4. Softgel Capsule Enteric Film Coating Formulation**

| | **Weight (g)** | **% Solids** |
|---|---|---|
| Enteric Polymer (Manucol LD) | 6.0 | 6.0 |
| KOLLICOAT IR | 3.0 | 3.0 |
| DI Water | 91.0 | |
| Total | 100.00 | 9.0 |

### EXAMPLE 3

### Enteric Performance of Enteric Film Coating with a Plasticizer

An aqueous enteric coating solution was prepared similar to the formulation of Example 2 but with higher solid content (see, Table 5). The formulation had a viscosity of 597 cps at 9.9% solids. It was coated onto S19 gelatin capsules (0.5" x 0.375" diameter). The resulting coating had a thickness of 54 microns and 5.4% weight gain. After coating, the capsules were tested for enteric performance according to the method described above. After 1 hour in SGF, 6/6 of the coated capsules remained intact. These results suggest that the combination of low viscosity alginate with low G content and film formation polymer when used with proper plasticizer is also able to provide an enteric film coating with adequate enteric performance.

**Table 5. Softgel Capsule Enteric Film Coating Formulation**

| | **Weight (g)** | **% Solids** |
|---|---|---|
| Enteric Polymer (Manucol LD) | 4.5 | 4.5 |
| Film Formation Polymer (KOLLICOAT IR) | 4.5 | 4.5 |
| Plasticizer (glycerol) | 0.9 | 0.9 |
| DI Water | 90.1 | |
| Total | 100.00 | 9.9 |

### EXAMPLE 4 (not according to the invention) Enteric Performance of Enteric Film Coating using Low G Content, Medium Viscosity Alginate

An aqueous enteric coating solution was prepared according to the formulation in Table 6. Manucol DH is a medium viscosity alginate with low G content. The formulation had a viscosity of 847 cps at 5.0% solids. S19 gelatin capsules (0.5" x 0.375" diameter) were coated with the formulation. The resulting coating had a thickness of 40 microns at 2.5% weight gain. After coating, the capsules were tested for enteric performance according to the method described above. All 6 of the coated capsules remained intact for 1 hour and 4/6 remained intact for 2 hours. These results suggest that the combination of medium viscosity alginate with low G content and film formation polymer is able to provide an enteric film coating with adequate enteric performance. Furthermore the amount of alginate used was able to be lowered due to the increased viscosity of the alginate.

**Table 6. Softgel Capsule Enteric Film Coating Formulation**

| | **Weight (g)** | **% Solids** |
|---|---|---|
| Enteric Polymer (Manucol DH) | 4.2 | 4.2 |
| Film Formation Polymer (KOLLICOAT IR) | 4.2 | 4.2 |
| Plasticizer (glycerol) | 1.7 | 1.7 |
| Antifoaming Agent (DOW Q7-2587) | 0.10 | 0.10 |
| DI Water | 89.8 | |
| Total | 100.00 | 10.2 |

### EXAMPLE 5

### Enteric Performance of Enteric Film Coating Using PEG8 Plasticizer after 6 Weeks Storage

An aqueous enteric coating solution was prepared according to the formulation in Table 7 and coated onto S23 Spring Valley gelatin fish oil capsules (1" x 0.33" diameter) at 8.3% gain. The capsules were then stored at room temperature for 6 weeks and tested for enteric performance according to the method described above. After 2 hour in SGF, 6/6 of the coated capsules remained intact. The coated soft gels were placed in SIF afterwards. All capsules were completely emptied after 8 minutes. The capsule shells were completely dissolved within 18 minutes. These results suggest that the enteric performance of the enteric film coating is stable after storage for 6 weeks.

**Table 7. Softgel Capsule Enteric Film Coating Formulation**

| | **Weight (g)** | **% Solids** |
|---|---|---|
| Enteric Polymer (Manucol LD) | 4.0 | 4.0 |
| Film Formation Polymer (HPMC K3 LV) | 3.6 | 3.6 |
| Plasticizer (PEG8) | 0.4 | 0.4 |
| DI Water | 92.0 | |
| Total | 100.00 | 8.0 |

### EXAMPLE 6

### Enteric Performance of Enteric Film Coating Using Low Viscosity Modified Starch

An aqueous enteric coating solution was prepared according to the formulation in Table 8 and resulted in a solution with 907 cps viscosity. PURE-COTE B792 is a modified food starch designed specifically for producing clear, flexible films. The formulation was coated onto S22 size fish oil capsules resulting in a 3% weight gain. The film coating is homogeneous and clear with excellent adhesion. This 3% weight gain coated capsules well passed the 1 hour disintegration time in SGF at 37°C. These results suggest that the combination of low viscosity alginate with low G content and a low viscosity modified plant starch when used with a plasticizer is able to provide an enteric film coating with adequate enteric performance without the use of an antifoaming agent.

**Table 8. Softgel Capsule Enteric Film Coating Formulation**

| | **Weight (g)** | **% Solids** |
|---|---|---|
| Enteric Polymer (Manucol LB) | 5.0 | 5.0 |
| Film Formation Polymer (PURE-COTE B792) | 3.4 | 3.4 |
| Plasticizer (glycerin) | 1.7 | 1.7 |
| DI Water | 89.9 | |
| Total | 100.00 | 10.1 |

### EXAMPLE 7

### Enteric Performance of Enteric Film Coating Using Low Viscosity Modified Pea Starch

An aqueous enteric coating solution was prepared according to the formulation in Table 9. LyCoat RS780 is a modified pea starch specifically developed for the aqueous film coating for immediate release. The formulation had viscosity of 780 cps. S22 size fish oil capsules were coated resulting in a 3% weight gain. The film coating was transparent and homogeneous with excellent adhesion to the gelatin capsule surfaces. Six of these capsules were tested for USP disintegration test. All 6 capsules passed the 1 hour time in SGF at 37°C. Similar results were obtained with LyCoat RS 720 material with slightly higher formulation viscosity (867 cps). These results suggest that the combination of low viscosity alginate with low G content and a low viscosity modified pea starch when used with a plasticizer is able to provide an enteric film coating with adequate enteric performance without the use of an antifoaming agent.

**Table 9. Softgel Capsule Enteric Film Coating Formulation**

| | **Weight (g)** | **% Solids** |
|---|---|---|
| Enteric Polymer (An alginate blend) | 5.0 | 5.0 |
| Film Formation Polymer (LyCoat RS780) | 3.4 | 3.4 |
| Plasticizer (glycerin) | 1.7 | 1.7 |
| DI Water | 89.9 | |
| Total | 100.00 | 10.1 |

### COMPARATIVE EXAMPLE 8

### Enteric Performance of an Enteric Coating Using Low G Alginate without a Film Formation Polymer

An aqueous coating solution was prepared according to the formulation in Table 10 and resulted in a formulation with 735cps. S23 fish oil capsules were coated with the formulation resulting in a 10.9% weight gain. In the standard USP disintegration test 2/6 of the coated capsules ruptured after only 30minutes and 4/6 of the coated capsules ruptured by 1 hour in SGF. Only 2/6 of the coated capsules remain intact at the 1 hour mark. These results suggest that low viscosity alginate with a low G content when used without a film formation polymer is unable to provide an enteric film coating with adequate enteric performance. Careful formulating with other ingredients is necessary to obtain proper functionalities.

**Table 10. Softgel Capsule Enteric Film Coating Formulation**

| | **Weight (g)** | **% Solids** |
|---|---|---|
| Enteric Polymer (Manucol LD) | 6.0 | 6.0 |
| Film Formation Polymer | 0.0 | 0.0 |
| Plasticizer (glycerin) | 1.2 | 1.2 |
| Antifoaming Agent | 0.0 | 0.0 |
| DI Water | 92.8 | |
| Total | 100.00 | 7.2 |

### COMPARATIVE EXAMPLE 9

### Enteric Performance of an Enteric Coating Using High G Alginate without a Film Formation Polymer

An aqueous coating solution was prepared according to the formulation in Table 10. Protonal GP1740 is a High G alginate used for specific gelling, thickening, and stabilizing applications. The formulation was coated to a fish oil capsules resulting in a 10.8% weight gain. After coating, the capsules were tested for enteric performance according to the method described above. After 15 minutes in SGF, 6/6 of the coated capsules were completely emptied. These results suggest that low viscosity alginate with a high G content when used without a film formation polymer is unable to provide an enteric film coating with adequate enteric performance.

**Table 11. Softgel Capsule Enteric Film Coating Formulation**

| | **Weight (g)** | **% Solids** |
|---|---|---|
| Enteric Polymer (Protonal GP1740) | 6.0 | 6.0 |
| Plasticizer (glycerin) | 1.2 | 1.2 |
| DI Water | 92.8 | |
| Total | 100.00 | 7.2 |

### COMPARATIVE EXAMPLE 10

### Enteric Performance of High G Alginate Polymer with Film Formation Polymer

An aqueous coating solution was prepared according to the formulation in Table 12. The High G content alginate Protonal GP1740 was used in combination with the film formation polymer HPMC E15. The formulation had a viscosity of 95 cps. The capsules, when coated resulted in a 11.6% weight gain, 6/6 of the coated capsules were completely cracked at 4 minutes in SGF. These results suggest that the combination of a high G content low viscosity alginate and a film formation polymer is unable to provide an enteric film coating with adequate enteric performance even coated very thick.

**Table 12. Softgel Capsule Enteric Film Coating Formulation**

| | **Weight (g)** | **% Solids** |
|---|---|---|
| Enteric Polymer (Protonal GP1740) | 3.0 | 3.0 |
| Film Formation Polymer 2 (HPMC E15) | 2.0 | 2.0 |
| Plasticizer (PEG 8) | 5.0 | 5.0 |
| Antifoaming Agent | 0.0 | 0.0 |
| DI Water | 90.0 | |
| Total | 100.00 | 10.0 |

### COMPARATIVE EXAMPLE 11

### Enteric Performance of High G Alginate Polymer Formulation

An aqueous coating solution was prepared according to the formulation in Table 13. The High G content alginate Manugel FB was used in combination with the film formation polymer HPMC E3LV. This formulation had a viscosity of 557 cps and coated to a S19 gelatin softgels (0.5" x 0.375" diameter) resulting in an 8.0% weight gain. With the standard USP disintegration test, 6/6 of the coated capsules were completely emptied after 32 minutes in SGF. These results again suggest that the combination of a high G content low viscosity alginate and a film formation polymer is unable to provide an enteric film coating with adequate enteric performance.

**Table 13. Softgel Capsule Enteric Film Coating Formulation**

| | **Weight (g)** | **% Solids** |
|---|---|---|
| Enteric Polymer (Manugel FB) | 4.2 | 4.2 |
| Film Formation Polymer 2 (HPMC E3LV) | 4.2 | 4.2 |
| Plasticizer (glycerin) | 2.5 | 2.5 |
| Antifoaming Agent | 0.1 | 0.1 |
| DI Water | 89.00 | |
| Total | 100.00 | 11.0 |

## Claims

1. A water soluble dry powder formulation useful for forming an aqueous enteric coating solution comprising an alginate and a film formation polymer, wherein:
the ratio of alginate to film formation polymer is 10: 1 to 1 :3 by weight,
the alginate is water soluble at 10% solids and has a G content of 20% to 40 % and a viscosity of 3-20 mPa*s (cps) at 1% solids (measured in water at 20°C+/-2°C using a Brookfield viscosimeter spindle #62 at 12 rpm),
the film formation polymer is water soluble at 10% solids, and
whereby when the aqueous enteric coating solution comprising the water soluble dry powder formulation is applied on an oral dosage form containing an active ingredient,
the active ingredient is not released into a medium of 0.1 N HCl at about 37°C for up to one hour, and
thereafter about 90% or more of the active ingredient is released within two hours in a phosphate buffer medium having pH 7.2 at about 37°C.

2. The water soluble dry powder formulation of claim 1, wherein the alginate is in monovalent salt form.

3. The water soluble dry powder formulation of any one of claims 1 or 2, wherein the film formation polymer is hydroxypropyl methylcellulose (HPMC) or a modified plant starch with a viscosity of 100 to 1000 mPa*s (cps) at 10% solids (measured in water at 20°C+/-2°C using a Brookfield viscosimeter spindle #62 at 12 rpm).

4. The water soluble dry powder formulation of any one of claims 1-3, wherein the film formation polymer is modified corn starch with a viscosity of 100 to 1000 mPa*s (cps) at 10% solids (measured in water at 20°C+/-2°C using a Brookfield viscosimeter spindle #62 at 12 rpm).

5. The water soluble dry powder formulation of any one of claims 1-4, wherein the water soluble dry powder formulation is substantially phthalate free.

6. The water soluble dry powder formulation of claims 1-5, wherein the alginate is sodium alginate or potassium alginate.

7. The water soluble dry powder formulation of claims 1-6, wherein said enteric polymer comprises alginate and said film formation polymer comprises at least one of a modified corn starch or pea starch with a viscosity of 100 to 1000 mPa*s (cps) at 10% solids (measured in water at 20°C+/-2°C using a Brookfield viscosimeter spindle #62 at 12 rpm).

8. An aqueous enteric coating solution useful for forming an enteric film coating comprising the water soluble dry powder formulation of any one of claims 1-7 dissolved in the aqueous enteric coating solution.

9. The aqueous enteric coating solution of claim 8, further comprising a plasticizer, and optionally an antifoaming agent, optionally the plasticizer is glycerin, and optionally the antifoaming agent is simethicone.

10. The aqueous enteric coating solution of any one of claims 8 or 9, wherein the aqueous enteric coating solution has a viscosity of 50 to 2000 mPa*s (cps) and optionally a viscosity of 100 to 1000 mPa*s (cps) or 100 to 800 mPa*s (cps) (measured in water at 20°C+/-2°C using a Brookfield viscosimeter spindle #62 at 12 rpm).

11. An enteric film coated oral dosage form comprising an active ingredient, wherein:
the oral dosage from is coated with the aqueous enteric coating solution of any one of claims 8-10.

12. The enteric film coated oral dosage form of claim 11, wherein:
the active ingredient contained in the enteric film coated oral dosage form is not released into the medium of 0.1 N HC1 at about 37°C for up to one hour, and
thereafter about 90% or more of the active ingredient is released within two hours in a phosphate buffer medium having pH 7.2 at about 37°C after six weeks storage at 20 degrees Celsius.

13. The enteric film coated oral dosage form of any one of claims 11 or 12, wherein the oral dosage form is a tablet or a soft capsule, optionally the active ingredient is a pharmaceutical, nutraceutical, veterinary, or food product, and optionally the active ingredient is fish oil, garlic, Probiotic, Lumbrokinase, S-Adenosyl Methionine (SAM-e), Nattokinase, a nonsteroidal anti-inflammatory drug, or iron supplement.

## Patentansprüche

1. Wasserlösliche Trockenpulverformulierung, die zur Herstellung einer wässrigen Magensaftresistente-Beschichtung-Lösung verwendbar ist, umfassend ein Alginat und ein Filmbindungspolymer, wobei:
das Verhältnis von Alginat zu Filmbindungspolymer 10:1 bis 1:3 nach Gewicht beträgt,
das Alginat auf einen Feststoffgehalt von 10 % wasserlöslich ist und einen G-Gehalt von 20 % bis 40 % und eine Viskosität von 3-20 mPa.s (cps) bei 1 % Feststoffgehalt (gemessen in Wasser bei 20 °C +/- 2 °C unter Verwendung einer Brookfield-Viskosimeter-Spindel #62 bei 12 U/min) aufweist,
das Filmbildungspolymer auf einen Feststoffgehalt von 10 % wasserlöslich ist und
wobei, wenn die wässrige Magensaftresistente-Beschichtungs-Lösung, die die wasserlösliche Trockenpulverformulierung umfasst, auf eine orale Darreichungsform, die einen Wirkstoff enthält, aufgebracht ist,
der Wirkstoff in ein Medium von 0,1 N HCl bei etwa 37 °C für bis zu einer Stunde nicht freigesetzt wird und
anschließend innerhalb von zwei Stunden etwa 90 % oder mehr des Wirkstoffs in einem Phosphatpuffermedium mit einem pH-Wert von 7,2 bei etwa 37 °C freigesetzt werden.

2. Wasserlösliche Trockenpulverformulierung gemäß Anspruch 1, wobei das Alginat in einwertiger Salzform vorliegt.

3. Wasserlösliche Trockenpulverformulierung gemäß einem der Ansprüche 1 oder 2, wobei das Filmbildungspolymer Hydroxypropylmethylcellulose (HPMC) oder eine modifizierte Pflanzenstärke mit einer Viskosität von 100 bis 1000 mPa.s (cps) bei 10 % Feststoffgehalt (gemessen in Wasser bei 20 °C +/- 2 °C unter Verwendung einer Brookfield-Viskosimeter-Spindel #62 bei 12 U/min) ist.

4. Wasserlösliche Trockenpulverformulierung gemäß einem der Ansprüche 1-3, wobei das Filmbildungspolymer modifizierte Maisstärke mit einer Viskosität von 100 bis 1000 mPa.s (cps) bei 10 % Feststoffgehalt (gemessen in Wasser bei 20 °C +/- 2 °C unter Verwendung einer Brookfield-Viskosimeter-Spindel #62 bei 12 U/min) ist.

5. Wasserlösliche Trockenpulverformulierung gemäß einem der Ansprüche 1-4, wobei die wasserlösliche Trockenpulverformulierung im Wesentlichen phthalatfrei ist.

6. Wasserlösliche Trockenpulverformulierung gemäß einem der Ansprüche 1-5, wobei das Alginat Natriumalginat oder Kaliumalginat ist.

7. Wasserlösliche Trockenpulverformulierung gemäß einem der Ansprüche 1-6, wobei das magensaftresistente Polymer Alginat umfasst und das Filmbildungspolymer wenigstens eines von einer modifizierten Maisstärke oder Erbsenstärke mit einer Viskosität von 100 bis 1000 mPa.s (cps) bei 10 % Feststoffgehalt (gemessen in Wasser bei 20 °C +/- 2 °C unter Verwendung einer Brookfield-Viskosimeter-Spindel #62 bei 12 U/min) umfasst.

8. Wässrige Magensaftresistente-Beschichtung-Lösung, die zur Herstellung einer magensaftresistenten Filmbeschichtung geeignet ist, umfassend die wasserlösliche Trockenpulverformulierung gemäß einem der Ansprüche 1-7 gelöst in der wässrigen Magensaftresistente-Beschichtung-Lösung.

9. Wässrige Magensaftresistente-Beschichtung-Lösung gemäß Anspruch 8, ferner umfassend einen Weichmacher und gegebenenfalls ein Antischaummittel, wobei der Weichmacher gegebenenfalls Glycerin ist und das Antischaummittel gegebenenfalls Simethicon ist.

10. Wässrige Magensaftresistente-Beschichtung-Lösung gemäß einem der Ansprüche 8 oder 9, wobei die wässrige Magensaftresistente-Beschichtung-Lösung eine Viskosität von 50 bis 2000 mPa.s (cps) und gegebenenfalls eine Viskosität von 100 bis 1000 mPa.s (cps) oder 100 bis 800 mPa.s (cps) (gemessen in Wasser bei 20 °C +/- 2 °C unter Verwendung einer Brookfield-Viskosimeter-Spindel #62 bei 12 U/min) aufweist.

11. Magensaftresistente filmbeschichtete orale Darreichungsform, die einen Wirkstoff umfasst, wobei:
die orale Darreichungsform mit der wässrigen Magensaftresistente-Beschichtung-Lösung gemäß einem der Ansprüche 8-10 beschichtet ist.

12. Magensaftresistente filmbeschichtete orale Darreichungsform gemäß Anspruch 11, wobei:
der in der magensaftresistenten filmbeschichteten oralen Darreichungsform enthaltene Wirkstoff in ein Medium von 0,1 N HCl bei etwa 37 °C für bis zu einer Stunde nicht freigesetzt wird und
anschließend nach sechs Wochen Lagerung bei 20 Grad Celsius etwa 90 % oder mehr des Wirkstoffs innerhalb von zwei Stunden in einem Phosphatpuffermedium mit einem pH-Wert von 7,2 bei etwa 37 °C freigesetzt werden.

13. Magensaftresistente filmbeschichtete orale Darreichungsform gemäß einem der Ansprüche 11 oder 12, wobei die orale Darreichungsform eine Tablette oder eine Weichkapsel ist, wobei der Wirkstoff gegebenenfalls ein pharmazeutisches, nutrazeutisches, tiermedizinisches oder Lebensmittelprodukt ist und der Wirkstoff gegebenenfalls Fischöl, Knoblauch, ein Probiotikum, Lumbrokinase, S-Adenosylmethionin (SAM-e), Nattokinase, ein nichtsteroides entzündungshemmendes Arzneimittel oder ein Eisenergänzungsmittel ist.

## Revendications

1. Formulation de poudre sèche soluble dans l'eau utile pour la formation d'une solution aqueuse de revêtement entérique comprenant un alginate et un polymère de formation de film,
le rapport d'alginate sur polymère de formation de film étant de 10 : 1 à 1 : 3 en poids,
l'alginate étant soluble dans l'eau à raison de 10 % de solides et possédant une teneur en G de 20 % à 40 % et une viscosité de 3 à 20 mPa.s (cps) à 1 % de solides (mesurée dans l'eau à 20 °C ± 2 °C à l'aide d'une tige #62 de viscosimètre Brookfield à 12 rpm),
le polymère de formation de film étant soluble dans l'eau à raison de 10 % de solides, et
la solution aqueuse de revêtement entérique comprenant la formulation de poudre sèche soluble dans l'eau étant appliquée sur une forme de dosage orale contenant un ingrédient actif,
l'ingrédient actif n'étant pas libéré dans un milieu de 0,1 N HCl à environ 37 °C pendant une durée allant jusqu'à une heure, et
ensuite environ 90 % ou plus de l'ingrédient actif étant libéré dans les deux heures dans un milieu de tampon phosphate possédant un pH de 7,2 à environ 37 °C.

2. Formulation de poudre sèche soluble dans l'eau selon la revendication 1, l'alginate étant sous forme de sel monovalent.

3. Formulation de poudre sèche soluble dans l'eau selon l'une quelconque des revendications 1 et 2,
le polymère de formation de film étant une hydroxypropylméthylcellulose (HPMC) ou un amidon végétal modifié doté d'une viscosité de 100 à 1 000 mPa.s (cps) à 10 % de solides (mesurée dans l'eau à 20 °C ± 2 °C à l'aide d'une tige #62 de viscosimètre Brookfield à 12 rpm).

4. Formulation de poudre sèche soluble dans l'eau selon l'une quelconque des revendications 1 à 3, le polymère de formation de film étant un amidon de maïs modifié doté d'une viscosité de 100 à 1 000 mPa.s (cps) à 10 % de solides (mesurée dans l'eau à 20 °C ± 2 °C à l'aide d'une tige #62 de viscosimètre Brookfield à 12 rpm).

5. Formulation de poudre sèche soluble dans l'eau selon l'une quelconque des revendications 1 à 4, la formulation de poudre sèche soluble dans l'eau étant sensiblement exempte de phtalates.

6. Formulation de poudre sèche soluble dans l'eau selon l'une quelconque des revendications 1 à 5, l'alginate étant un alginate de sodium ou un alginate de potassium.

7. Formulation de poudre sèche soluble dans l'eau selon l'une quelconque des revendications 1 à 6, ledit polymère entérique comprenant un alginate et ledit polymère de formation de film comprenant au moins l'un parmi un amidon de maïs modifié et un amidon de pois modifié, doté d'une viscosité de 100 à 1 000 mPa.s (cps) à 10 % de solides (mesurée dans l'eau à 20 °C ± 2 °C à l'aide d'une tige #62 de viscosimètre Brookfield à 12 rpm).

8. Solution aqueuse de revêtement entérique utile pour la formation d'un revêtement de film entérique comprenant la formulation de poudre sèche soluble dans l'eau selon l'une quelconque des revendications 1 à 7 dissoute dans la solution aqueuse de revêtement entérique.

9. Solution aqueuse de revêtement entérique selon la revendication 8, comprenant en outre un plastifiant, et éventuellement un agent antimousse, éventuellement le plastifiant étant la glycérine, et éventuellement l'agent antimousse étant la siméthicone.

10. Solution aqueuse de revêtement entérique selon l'une quelconque des revendications 8 et 9, la solution aqueuse de revêtement entérique possédant une viscosité de 50 à 2 000 mPa.s (cps) et éventuellement une viscosité de 100 à 1 000 mPa.s (cps) ou de 100 à 800 mPa.s (cps), (mesurée dans l'eau à 20 °C ± 2 °C à l'aide d'une tige #62 de viscosimètre Brookfield à 12 rpm).

11. Forme de dosage orale revêtue par un film entérique comprenant un ingrédient actif,
la forme de dosage orale étant revêtue avec la solution aqueuse de revêtement entérique selon l'une quelconque des revendications 8 à 10.

12. Forme de dosage orale revêtue par un film entérique selon la revendication 11,
l'ingrédient actif contenu dans la forme de dosage orale revêtue par un film entérique n'étant pas libéré dans un milieu de 0,1 N HCl à environ 37 °C pendant une durée allant jusqu'à une heure, et
ensuite environ 90 % ou plus de l'ingrédient actif étant libéré dans les deux heures dans un milieu de tampon phosphate possédant un pH de 7,2 à environ 37 °C après six semaines de stockage à 20 degrés Celsius.

13. Forme de dosage orale revêtue par un film entérique selon l'une quelconque des revendications 11 et 12, la forme de dosage orale étant un comprimé ou une capsule souple, éventuellement l'ingrédient actif étant un produit pharmaceutique, nutraceutique, vétérinaire ou alimentaire, et éventuellement l'ingrédient actif étant de l'huile de poisson, de l'ail, un Probiotique, de la Lumbrokinase, de la S-adénosyl-méthionine (SAM-e), de la Nattokinase, un médicament anti-inflammatoire non stéroïdien, ou un supplément de fer.
